# EUROPEAN PATENT APPLICATION

(11) **EP 2 243 440 A1**
(43) Date of publication of application: **27.10.2010**
(21) Application number: 10157090.1
(22) Date of filing: 19.03.2010
(51) Int. Cl.: A61C 3/00, B25G 1/10

(54) **Hand instrument for manual dental care**

(30) Priority: 23.03.2009 FI 20090113
(71) Applicant: LM-Instruments OY, 21601 Parainen (FI)
(72) Inventor: Luoto, Toni, FI-21600, Parainen (FI)
(74) Representative: Salomäki, Juha Kari Ensio

(57) **Abstract**

The invention relates to a hand-held instrument manually used in dental care. Most typically, hand-held instruments related to the invention are used in connection with tartar scaling and when modelling tooth filling material in the patient's mouth. The hand-held instrument according to the invention comprises a substantially elongated handle (2) at least one end of which is arranged with a head part (3, 3') substantially parallel with the central axis of the handle (2) and, in the instrument, the central axis of the handle (2) is arranged substantially curved for its whole length or for at least a portion of its length.

## Description

### FIELD OF THE INVENTION

The invention relates to a hand-held instrument manually used in dental care. Most typically, hand-held instruments related to the invention are used in connection with tartar scaling and when modelling tooth filling material in the patient's mouth.

### BACKGROUND OF THE INVENTION

Commonly used hand-held instruments according to prior art comprise a substantially elongated handle part, at least one end of which is typically arranged with a head part protruding substantially in the direction of the central axis of the handle. The head part used in such instruments is usually bent at least at one point in order to facilitate the instrument reaching the target being operated on in the patient's mouth. Various sharply bent heads have been developed for use e.g. in the back teeth area often comprising targets difficult to reach. On the other hand, heads intended for working in the front teeth area can be more gently bent.

Although various shapes of head parts known from prior art can facilitate work on tooth surfaces being more difficult to reach, likewise as in the case of patients whose mouth opening is smaller than normal (e.g. rheumatic patients), it is still possible that problems related, inter alia, to the accuracy and strenuousness of the work occur when using them. When removing tartar from the back teeth area, for instance, it can be difficult, on the one hand, to produce sufficient force and, on the other hand, to provide the optimal pull direction of the instrument head for removing tartar. It is also possible that the handling of the instrument is hindered by the dental arch located vertically on the opposite side with respect to the target being operated on, which the instrument handle may tend to touch. Furthermore, when working on the lingual surfaces of lower incisives, the patient's upper teeth, lips or nose can be in the way of the instrument handle, especially if the lower incisives are pointing inwards. In addition, the handle of the prior-art elongated instrument, the surface of which possibly comprises some shapes and the central axis of which is substantially straight, can impede the visibility of the target area.

### BRIEF DESCRIPTION OF THE INVENTION

Although problems of the above kind have in some ways been minimized by developing different variations of head-part bendings, the purpose of this invention is to further diminish problems of the above kind and to provide a relatively cost-effectively manufacturable, easy-to-use and ergonomic instrument which is especially applicable to work on tooth surfaces difficult to reach. An example of such is the removal of tartar from the lingual surfaces of inward-pointing lower incisives and, on the other hand, from the distal surfaces of the rearmost molars. The instrument according to the invention provided with a suitable head part is also applicable to be used e.g. for modelling tooth filling material and for so-called tapping.

The instrument according to the invention is characterized by what is stated in the characterizing part of claim 1 enclosed. Some preferable embodiments of the invention are characterized by what is stated in the enclosed dependent claims.

It is thus essential to the invention that the central axis of the instrument handle is arranged curved for at least a portion of its length. In many situations, in which the target being operated on is located in a place difficult to reach, the curved handle can facilitate the handling of the instrument and, depending on the case, to increase the effectiveness, accuracy and speed of working and/or the force applicable to the target being operated on. The use of the instrument according to the invention enables a good care result due to, on the one hand, the reach of the instrument and, on the other hand, the good visibility of the target area.

### BRIEF DESCRIPTION OF THE DRAWINGS

Next, the invention will be described in more detail also with reference to the enclosed figures of which
- Fig. 1: presents a side view of one embodiment of an instrument according to the invention,
- Fig. 2: presents a side view of a second embodiment of the instrument according to the invention and
- Fig. 3: presents a third embodiment of the instrument according to the invention.

### DETAILED DESCRIPTION OF THE INVENTION

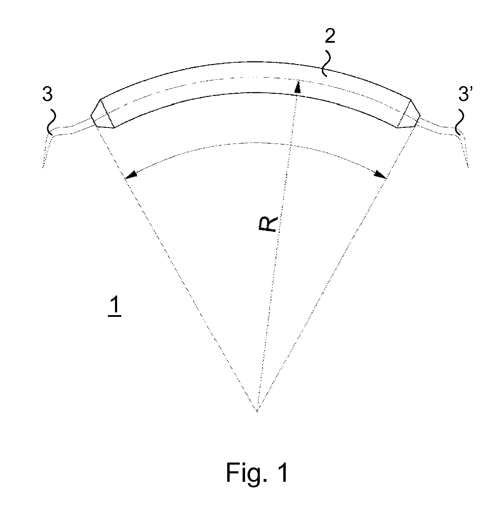

Fig. 1 presents a side view of one instrument 1 according to the invention. The instrument 1 according to Fig. 1 comprises a handle 2 having a substantially curved central axis and head parts 3, 3' extending from the ends of the handle. A radius of curvature R of the central axis of the handle 2 is constant in the embodiment according to Fig. 1.

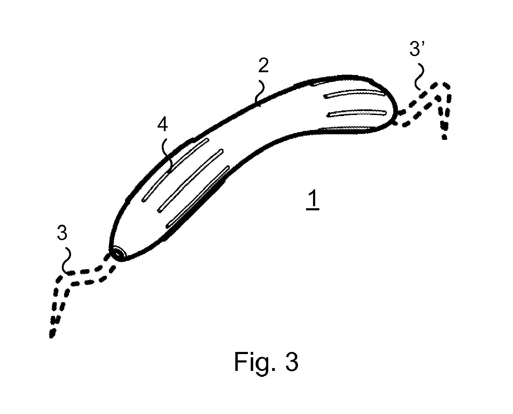

In Fig. 1, the surface of the instrument handle 2 is even and its diameter is constant substantially for the whole length of the handle 2. However, the diameter of the handle 2 of the instrument 1 can also vary at its various points, and the shape of the surface can be other than the one in the embodiment shown in Fig. 1. The handle 1 can comprise e.g. elongated projections 4 substantially parallel with the central axis of the handle 2, shown in Fig. 3, the purpose of which is to improve the grip of the handle 2. Especially, the diameter of the handle 2 can be arranged to uniformly increase when moving from the middle of the handle 2 in the direction of the head part 3, 3' such that the diameter first increases and then again decreases in the immediate proximity of the end of the handle 4. Such variation in the diameter of the handle 2 is an advantageous arrangement considering the usability and ergonomics of the instrument 1.

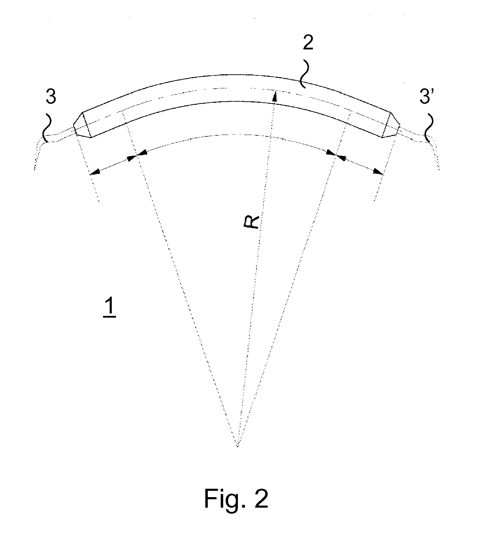

Preferable embodiments of the invention also include an arrangement in which the central axis of the handle 2 is substantially curved in the middle section of the handle 2 but straight close to the end of the handle 2 - or the ends of the handle 2 (Fig. 2). The arrangement in question can be advantageous considering the manufacture of the instrument and also its durability in use, especially if the head part 3, 3' is of harder material than the handle 2 and it is attached to the handle 2 such that the extension of the head part 3, 3' extends within the handle 2. The length of such a straight section of the central axis at the end or ends of the handle 2 can be e.g. about 15 mm. According to one preferable embodiment of the invention, such a straight section extends tangentially from the curved section of the central axis.

In the embodiments presented above, the total length of the central axis of the handle 2 is preferably e.g. of the order of 110 mm. On the other hand, the radius of curvature R of the central axis of the handle is in some preferable embodiments of the invention of the order of 100-150 mm.

Above embodiments of the invention were described in which both ends of the handle 2 is arranged with an actual head part 3, 3' used for working. Without diverging from the basic idea of the invention, the instrument can also be realized such that only the first end of the handle 2 includes the head part 3. Then according to a preferable embodiment of the invention, the central axis of the handle 2 can be arranged straight already starting substantially in the middle of the handle, i.e. such that the central axis of the handle 2 is curved for at least a portion of the length substantially in the half of the handle 2 on the side of the head part 3, and substantially the central axis of the other half of the handle 2 is substantially straight. Similarly, the total length of the central axis of the handle 2 can then be arranged longer than the above said about 110 mm, such as at least 120 mm or 140 mm, which in some working situations, and especially considering persons with large hands, can enhance having a firm grip of the instrument.

It is evident to those skilled in the art that the invention is not limited to the above examples, but its different embodiments can vary within the scope of the claims presented next. Thus, e.g. the head part 3, 3' of the instrument can be any head part 3, 3' intended for the removal of tartar or the modelling of filling material but also for diagnosis within the patient's mouth (a probe), whereby the handle 2 according to the invention can facilitate in creating a contact between the head part 3 and a difficult-to-reach tooth surface.

## Claims

1. A hand-held instrument used for manual dental care, which comprises a substantially elongated handle (2), one end or both ends of which is arranged with a head-part (3, 3') extending substantially from the central axis of the handle (2), **characterized in that** the central axis of the handle (2) is arranged substantially curved for its whole length or for at least a portion of its length.

2. A hand-held instrument according to claim 1, **characterized in that** the central axis of the handle (2) is curved substantially of its middle section and substantially straight at least at the first end of the handle (2) or at both ends of the handle (2).

3. A hand-held instrument according to claim 2, **characterized in that** said straight section of the central axis of the handle (2) is about 15 mm long.

4. A hand-held instrument according to claim 2 or 3, **characterized in that** said straight section of the central axis of the handle (2) tangentially converges with said curved section of the central axis.

5. A hand-held instrument according to any one of claims 1-4, **characterized in that** a radius of curvature R of the curvature of the central axis of said handle (2) is of the order of 100-150 mm.

6. A hand-held instrument according to any one of claims 1-5, **characterized in that** the length of the central axis of said handle (2) is about 110 mm.

7. A hand-held instrument according to any one of claims 1-6, **characterized in that** only the first end of the handle (2) is arranged with the head part (3), and that the central axis of the handle (2) is curved for at least a portion of the length substantially in the half of the handle (2) on the side of the head part (3), and substantially the central axis of the other half of the handle (2) is substantially straight.

8. A hand-held instrument according to claim 7, **characterized in that** the length of the central axis of the handle (2) is at least 120 mm or at least 140 mm.

9. A hand-held instrument according to any one of claims 1-8, **characterized in that** the diameter of the handle (2) is arranged to uniformly increase when moving from the middle of the handle (2) in the direction of the head part (3, 3') such that the diameter first increases and then again decreases in the immediate proximity of the end of the handle (4).
